# EUROPÄISCHE PATENTSCHRIFT

(11) **EP 0 386 628 B1**
(45) Veröffentlichungstag und Bekanntmachung des Hinweises auf die Patenterteilung: **23.06.1993**
(21) Anmeldenummer: 90103989.1
(22) Anmeldetag: 01.03.1990
(51) Int. Cl.: C07D 471/06, C07D 209/42

(54) **Verfahren zur Herstellung von Indolcarbonsäurederivaten**
Method for the preparation of indolcarboxylic acid derivatives
Procédé de préparation de dérivés de l'acide indolecarboxylique

(30) Priorität: 08.03.1989 DE 3907388
(43) Veröffentlichungstag der Anmeldung: 12.09.1990
(73) Patentinhaber: Kali-Chemie Pharma GmbH, D-30173 Hannover (DE)
(72) Erfinder: Mätzel, Uwe, D-3167 Burgdorf (DE); Heitmann, Walter, Marietta, GA 30068 (US)
(74) Vertreter: Lauer, Dieter, Dr.

(56) Entgegenhaltungen:
- EP-A- 0 139 584
- EP-A- 0 322 016
- US-A- 4 015 005
- CHEMICAL ABSTRACTS, Band 83, Nr. 17, 27. Oktober 1975 Columbus, Ohio, USA ALEMANY, A. "Enzyme inhibi- tors. X. Preparation and in vitro study of N2-substituted 2-(N methylindolyl) and 2-(N-benzylindolyl) carbohy- drazides as monoamineoxidase inhibitors" Seite 487, Spalte 2, Zusammenfassung-Nr. 147 368y
- CHEMICAL ABSTRACTS, Band 68, Nr. 3, 15. JUnner 1968 Columbus, Ohio, USA N. S. VUL'FSON et al. "Mass spectrometric study of hetero-cyclic compounds. IV. Fragmentation routes and some formyl- and carbalkoxy-in- doles" Seite 1154, Spalte 1, Zusammenfassung-Nr. 12 274j
- Journal of Heterocyclic Chemistry 11, (3), 387-393 (1974)

## Beschreibung

Die vorliegende Erfindung betrifft die Herstellung von als Zwischenprodukte in der pharmazeutischen Industrie geeigneten Indolcarbonsaurederivaten aus Anilinderivaten.

Indolcarbonsäurederivate können verwendet werden als Zwischenprodukte zur Herstellung von pharmazeutisch verwendbaren Wirkstoffen. Beispielsweise können Indolcarbonsäurederivate mit bicyclischen Alkaloidresten vom Strukturtyp der 8-Aza-bicyclo-[3.2.1]-octane, beispielsweise mit Tropin oder mit 3-Amino-8-methyl-8-azabicyclo-[3.2.1]-octan zu pharmakologischen Wirkstoffen mit 5-HT₃-antagonistischen Eigenschaften, welche zur Behandlung von Serotonin induzierten gastrointestinalen Beschwerden geeignet sind, umgesetzt werden (siehe EP 0 189 002 A2). Insbesondere sind aus 1,7-anellierten Indolcarbonsäurederivaten und den vorstehend genannten bicyclischen Alkaloidresten erhältliche pharmakologische Wirkstoffe starke und selektive Antagonisten von neuronalen 5 HT-Rezeptoren, welche zur Behandlung von durch Überstimulation dieser Rezeptoren induzierten Beschwerden geeignet sind (siehe EP 0 322 016 A1). Durch Umsetzung von 1,7-anellierten 1H-Indol-2-carbonsäurederivaten mit 3-Amino-1,4-benzodiazepinderivaten können pharmakologische Wirkstoffe mit CCK-antagonistischen Eigenschaften erhalten werden.

Indolcarbonsäurederivate können unter anderem durch Umsetzung von Phenylhydrazinderivaten mit Brenztraubensäurederivaten hergestellt werden. Die hierzu benötigten Phenylhydrazinderivate müssen ihrerseits aus entsprechenden Phenylnitrosaminderivaten gewonnen werden. Letztere werden durch Nitrierung von Anilinderivaten hergestellt.

Aufgabe der Erfindung ist es, ein Verfahren zur Herstellung von Indolcarbonsäurederivaten ausgehend von Anilinderivaten zu entwickeln, welches die Nachteile der vorstehend skizzierten Herstellungsweise vermeidet.

Es wurde nun ein Verfahren gefunden, in welchem Anilinderivate in einem Eintopfverfahren ohne Isolierung der gebildeten Zwischenprodukte direkt zu Indolcarbonsäurederivaten umgesetzt werden können.

Gegenstand der Erfindung ist ein Verfahren zur Herstellung von Indolcarbonsäurederivaten der allgemeinen Formel I 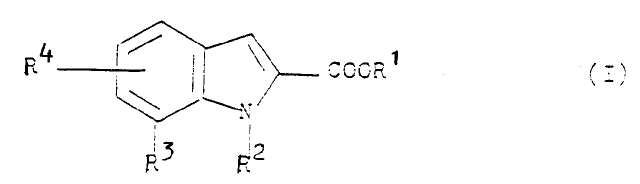
worin
- R¹: Wasserstoff oder niederes Alkyl bedeutet,
- R²: niederes Alkyl oder eine gegebenenfalls durch niederes Alkyl, Halogen oder niederes Alkoxy substituierte carbocyclische Aryl- oder Arylniederalkylgruppe bedeutet, und
- R³: Wasserstoff, niederes Alkyl, niederes Alkoxy oder eine gegebenenfalls durch niederes Alkyl, Halogen oder niederes Alkoxy substituierte carbocyclische Aryl-oder Arylniederalkylgruppe bedeutet, oder
- R² und R³: gemeinsam eine gegebenenfalls durch niederes Alkyl substituierte Alkylenkette mit 2 bis 4 Kohlenstoffatomen bilden, an welche gegebenenfalls ein 5-6-gliedriger carbocyclischer Ring anelliert sein kann, oder R² und R³ gemeinsam eine -X-CH₂-CH₂-Kette, worin X an den Phenylring des Indolgerüstes gebunden ist und Sauerstoff oder Schwefel bedeutet, bilden,
- R⁴: Wasserstoff, niederes Alkyl, niederes Alkoxy, Halogen oder Trifluormethyl bedeutet,

dadurch gekennzeichnet, daß man
a) Anilinderivate der allgemeinen Formel II 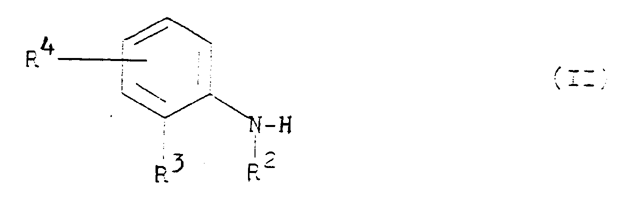 worin R² , R³ und R⁴ obige Bedeutung besitzen, in einer flüssigen organischen Säure oder in einem eine bezogen auf die Verbindung der Formel II mindestens äquivalente Menge einer Säure und gegebenenfalls ein mit Wasser mischbares organisches Lösungsmittel enthaltenden wäßrigen Medium mit Natriumnitrit bei Temperaturen zwischen 0 und 30 °C zu dem entsprechenden Nitrosamin umsetzt,
b) der unter a) erhaltenen nitrosaminhaltigen Reaktionslösung gegebenenfalls unter Zusatz eines mit Wasser mischbaren organischen Lösungsmittels eine bezogen auf die Verbindung der Formel II mindestens 2-fach molare Menge an metallischem Zink zusetzt und gegebenenfalls weitere Säure zusetzt, bis eine zur Reduktion des Nitrosamins zum entsprechenden Hydrazin ausreichende Säuremenge vorliegt, wobei das Zink in die bereits die gesamte Säuremenge enthaltende Lösung oder die Säure nach Zugabe des Zink unter Kühlung so langsam zugesetzt werden, daß in der Reaktionslösung eine Temperatur Reaktionslösung eine Temperatur zwischen 0 und 30 °C eingehalten wird, und das Reaktionsgemisch bei dieser Temperatur für eine zur Reduktion des Nitrosamins zu dem entsprechenden Hydrazin ausreichenden Zeit reagieren läßt, und gegebenenfalls weitere Säure zusetzt, um die Umsetzung eines in dem Reaktionsgemisch vorhandenen Überschuß an metallischem Zink zu vervollständigen,
c) die unter b) erhaltene hydrazinhaltige Reaktionslösung mit einem Brenztraubensäureniederalkylesters bei Tempe-Temperaturen zwischen Raumtemperatur und 120 °C umsetzt und gewünschtenfalls anschließend die gebildeten Indolcarbonsäureesterderivate der Formel Ia 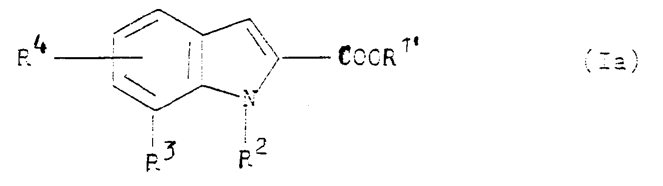 worin R² und R³ obige Bedeutung besitzen und R¹' niederes Alkyl bedeutet, zu der freien Säure hydrolysiert.

Das erfindungsgemäße Verfahren eignet sich insbesondere zur Herstellung von Indol-2-carbonsäurederivaten und von 4H-Pyrrolo[3,2,1-ij]-5,6-dihydrochinolin-2-carbonsäurederivaten.

Nach dem erfindungsgemäßen Verfahren wird das Ausgangsanilinderivat der Formel II zunächst in saurem Medium mit Natriumnitrit umgesetzt. Dabei bildet sich in der Reaktionslösung das der Verbindung der Formel II entsprechende Nitrosamin der Formel III

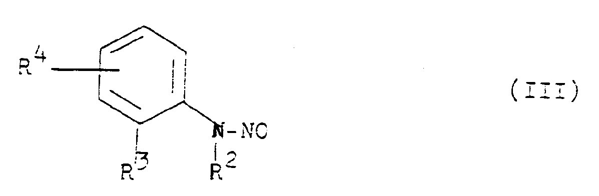

worin R² , R³ und R⁴ obige Bedeutung besitzen. Die Umsetzung erfolgt in saurem Medium, das heißt, der pH-Wert des Mediums sollte unter pH = 4 liegen. Das Anilinderivat der Formel II kann zum Beispiel in einer organischen Säure gelöst werden oder in einem Säure/Wasser-Gemisch, dem gegebenenfalls noch ein weiteres mit Wasser mischbares organisches Lösungsmittel zugesetzt werden kann, ganz oder teilweise gelöst werden und diese Reaktionslösung dann unter Kühlung mit einer wäßrigen Natriumnitritlösung versetzt werden. Zweckmäßig wird eine bezogen auf die Verbindung der Formel II äquimolare Menge oder ein nur geringer (bis zu 5 %) Überschuß an Natriumnitrit eingesetzt. Als Säuren kommen organische und anorganische mit Wasser mischbare Säuren in Frage, welche stark genug sind, um mit metallischem Zink unter Bildung von nascierendem Wasserstoff zu reagieren. Die Säurestärke kann je nach Art und Stabilität der Ausgangsaniline und der daraus intermediär zu bildenden Phenylhydrazinderivate variieren. Als geeignete organische Säuren seien gegebenenfalls durch Halogen oder Trifluormethyl substituierte niedere aliphatische Carbonsäuren genannt, beispielsweise Essigsäure oder Halogenessigsäuren. Als anorganische Säuren kommen beispielsweise Halogenwasserstoffsäuren oder Schwefelsäure in Frage. Bevorzugt werden Essigsäure, wäßrige Salzsäure oder deren Gemische verwendet.

Zur Verbesserung der Löslichkeit kann dem Reaktionsgemisch gewünschtenfalls bei der Umsetzung mit Natriumnitrit oder auch erst später bei der Reduktion mit metallischem Zink ein weiteres mit Wasser mischbares unter den Reaktionsbedingungen inertes organisches Lösungsmittel zugesetzt werden. Hierzu eignen sich insbesondere niedere Alkylalkohole. Flüssige organische Säuren können selbst auch als Lösungsmittel dienen. Ein Alkoholzusatz kann insbesondere dann zweckmäßig sein, wenn der Säuregehalt des Reaktionsgemisches überwiegend aus wäßrigen anorganischen Säuren gebildet wird. Die Umsetzung mit dem Natriumnitrit findet bei Temperaturen zwischen 0 und 30 °C, vorzugsweise zwischen 5 und 20 °C, statt. Die Reaktionszeit kann zwischen ca. 30 min. und zwei Stunden betragen. Die Konzentration des Ausgangsanilinderivates in der Reaktionslösung ist für den Verfahrensablauf nicht wesentlich und kann je nach Löslichkeit der Verbindungen variiert werden. Das Anilinderivat kann in der Reaktionslösung vollständig gelöst oder auch nur zum Teil gelöst und zum Teil suspendiert vorliegen. Als zweckmäßig haben sich 0,5 bis 2-molare, insbesondere ca. 1 bis 1,5-molare, Lösungen des Ausgangsstoffes erwiesen.

Zur Reduktion des Nitrosamins wird dem Reaktionsgemisch metallisches Zink, vorzugsweise metallisches Zinkpulver zugesetzt. Es wird eine auf die Ausgangsverbindung der Formel II bezogen mindestens 2-fach molare Menge an metallischem Zink, vorzugsweise ein Überschuß beispielsweise eine 2 bis 5-fach, insbesondere 2,5 bis 4-fach, molare Menge eingesetzt.

Falls das Reaktionsgemisch nach Beendigung der Nitrosamierung keine für die nachfolgende Reduktion des Nitrosamins zum Hydrazin ausreichende Menge Säure enthält, wird dem Reaktionsgemisch außerdem mindestens so viel weitere Säure zugesetzt, daß eine ausreichende Säuremenge, das ist eine Menge von mindestens 4, zweckmäßigerweise mindestens 5 Äquivalenten Säuren bezogen auf die Ausgangsverbindung, erreicht wird. Bei Verwendung von überwiegend anorganischen Säuren eignen sich beispielsweise Säuremengen von 5 bis 9 Äquivalenten bezogen auf die Ausgangsverbindungen Formel II. Sofern organische Säuren auch als Lösungsmittel mit eingesetzt sind, können selbstverständlich von diesen auch weitaus größer Mengen in dem Reaktionsgemisch vorhanden sein.

Hierbei kann so vorgegangen werden, daß dem Reaktionsgemisch zunächst die gesamte Säuremenge zugegeben und dann das Zink portionsweise unter Kühlung, vorzugsweise Eiskühlung, so langsam zugesetzt wird, daß in dem Reaktionsgemisch eine Temperatur zwischen 0 und 30 °C, vorzugsweise 5 bis 25 °C, eingehalten wird. Es kann aber auch erst das Zink zugegeben werden und dann die restliche Säure so langsam unter Kühlung zugegeben werden, daß die Temperatur 30 °C nicht überschreitet. Die Zugabe von Zink und Säure zu dem Reaktionsgemisch kann beispielsweise über einen Zeitraum von 1 bis 3 Stunden verteilt erfolgen. Nach Beendigung der Zugabe wird das Reaktionsgemisch zweckmäßig noch eine weitere Zeit vorzugsweise bei Raumtemperatur gerührt, um eine vollständige Überführung des Nitrosamins in das entsprechende Phenylhydrazin der Formel IV 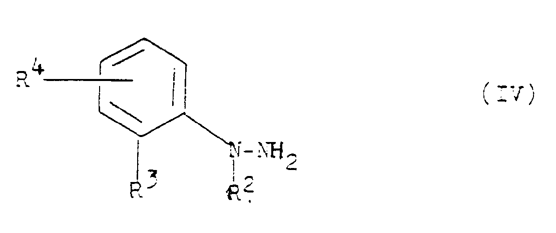
worin R², R³ und R⁴ obige Bedeutung besitzen, zu erreichen. Es kann vorteilhaft sein, um eine vollständige Umsetzung eines in dem Reaktionsgemisch vorhandenen Überschusses an metallischem Zink zu gewährleisten und gewünschtenfalls die entstehenden Zinksalze vollständig zu lösen, dem Reaktionsgemisch nochmals Säure, beispielsweise wäßrige Salzsäure, zuzusetzen.

Anschließend wird das Reaktionsgemisch mit einem Brenztraubensäureniederalkylester der Formel V 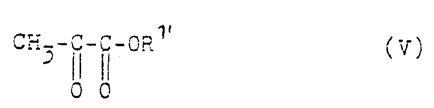
worin R¹' obige Bedeutung besitzt, umgesetzt. Es kann eine bezogen auf die Verbindung der Formel II äquimolare Menge oder auch ein bis zu 3-facher Überschuß an Brenztraubensäureniederalkylester, insbesondere Brenztraubensäureäthylester, eingesetzt werden. Die Umsetzung kann bei Temperaturen zwischen Raumemperatur und 120 °C, vorzugsweise bei Siedetemperatur, des Reaktionsgemisches durchgeführt werden. Die Reaktionszeit kann beispielsweise zwischen 0,5 und 2 Stunden betragen. Bei Zugabe des Brenztraubensäureesters zu dem die Phenylhydrazinverbindung der Formel IV enthaltenden Reaktionsgemisch entsteht intermediär die Hydrazonverbindung der Formel VI 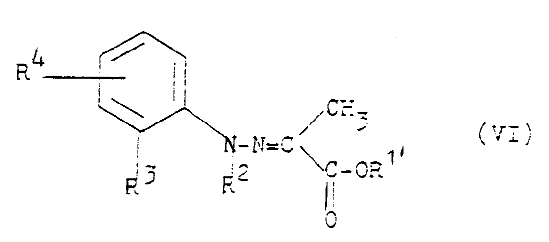
worin R¹', R² , R³ und R⁴ obige Bedeutung besitzen, welche unter den Reaktionsbedingungen weiter zu dem Ester der Formel Ia kondensiert.

Die Verbindungen der Formel Ia können auf an sich bekannte Weise aus dem Reaktionsgemisch isoliert und gereinigt werden. Beispielsweise können die Verbindungen mit einem Halogenkohlenwasserstoff, vorzugsweise Dichlormethan aus dem Reaktionsgemisch extrahiert werden. Das nach Abziehen des Lösungsmittels erhaltene Rohprodukt kann gewünschtenfalls auf an sich bekannte Weise chromatographisch gereinigt werden.

Die Ester der Formel Ia können in an sich bekannter Weise zu den entsprechenden Säuren hydrolysiert werden.

Das erfindungsgemäße Verfahren besitzt gegenüber den bisher zur Herstellung von Indolcarbonsäurederivaten aus entsprechenden Anilinderivaten üblichen Verfahren den Vorteil, daß die über mehrere Zwischenprodukte führende Umsetzung in einem Eintopfverfahren so geführt wird, daß keine Isolierung von Zwischenprodukten notwendig ist. Es kann somit das Arbeiten mit Nitrosamin- und Phenylhydrazinderivaten vermieden werden.

Verbindungen der Formel II sind bekannt oder können nach an sich bekannten Methoden hergestellt werden.

Die nachfolgenden Beispiele sollen die Erfindung näher erläutern.

### Beispiel 1:

### 4H-Pyrrolo[3,2,1-ij]-5,6-dihydrochinolin-2-carbonsäureäthylester.

150 g 1,2,3,4-Tetrahydrochinolin wurden in 1,25 l Eisessig gelöst. Zu der Lösung wurde unter Kühlung im Eisbad auf ca. 15 °C Innentemperatur eine Lösung von 80 g Natriumnitrit in 300 ml Wasser gegeben und das Reaktionsgemisch wurde 45 Minuten lang weiter gerührt. Zu der das gebildete N-Nitroso-1,2,3,4-Tetrahydrochinolin enthaltenden Reaktionslösung wurden während 1,5 Stunden portionsweise 300 g Zinkstaub zugegeben, wobei das Reaktionsgemisch durch Kühlen im Eisbad auf einer Innentemperatur von 15-30 °C gehalten wurde. Abschließend wurden 1,75 l Wasser und 1,25 l 32 %-ige wäßrige Salzsäure zu dem Gemisch gegeben und weitere 1,5 Stunden gerührt. Zu dem das gebildete N-Amino-1,2,3,4-tetrahydrochinolin und das Zinksalz enthaltenden saurem Reaktionsgemisch wurden 130 g Brenztraubensäureäthylester gegeben und das Gemisch 1 1/2 Stunden am Rückfluß erhitzt und anschließend weitere 16 Stunden stehen gelassen. Hierbei wurde das intermediär gebildete Hydrazon sofort in situ zu 4H-Pyrrolo[3,2,1,-ij]-5,6-dihydrochinolin-2-carbonsäureäthylester kondensiert. Zur Aufarbeitung wurde das Reaktionsgemisch zweimal mit insgesamt 5 l Dichlormethan extrahiert, die Dichlormethanextrakte vereinigt, zweimal mit insgesamt 1 l Wasser gewaschen, über Natriumsulfat getrocknet und eingeengt. Es wurden 280 g roher 4H-Pyrrolo[3,2,1-ij]-5,6-dihydrochinolin-2-carbonsäureäthylester erhalten, welche durch Chromatographie an Kieselgel unter Verwendung von Dichlormethan als Eluens gereinigt wurden. Es wurden 151, 8 g der gereinigten Titelverbindung mit einen Schmelzpunkt von 70-72 °C erhalten.

### Beispiel 2:

### 4H-Pyrrolo[3,2,1-ij]-5,6-dihydrochinolin-2-carbonsäure.

39 g 4H-Pyrrolo[3,2,1-ij]-5,6-dihydrochinolin-2-carbonsäureäthylester wurden in 40 ml Äthanol gelöst und diese Lösung wurde bei Raumtemperatur zu einer Lösung von 11,3 g Kaliumhydroxid in einem Gemisch aus 20 ml Wasser und 145 ml Äthanol gegeben. Das Reaktionsgemisch wurde 90 min. bei Raumtemperatur gerührt und dann auf 10 °C abgekühlt. Der ausgefallene Feststoff wurde abgesaugt und dreimal mit je 30 ml Äthanol gewaschen. Die Mutterlauge wurde auf die Hälfte eingeengt und der dabei ausfallende Feststoff wird ebenfalls abgetrennt und mit Äthanol gewaschen.

Der gesamte Feststoff wurde nun in 150 ml Wasser gelöst und daraus die Titelverbindung durch Ansäuern der Lösung mit konzentrierter Salzsäure auf pH 1 bis 2 gefällt. Die ausgefallene Säure wurde abgetrennt, dreimal mit je 40 ml Wasser gewaschen und bei 60 °C getrocknet. Es wurden 32,4 g der Titelverbindung mit einem Schmelzpunkt von 212-213 °C (Z) erhalten.

### Beispiel 3:

### 4H-Pyrrolo[3,2,1-ij]-5,6-dihydrochinolin-2-carbonsäureäthylester.

40 g Tetrahydrochinolin wurden bei Raumtemperatur in einer Mischung aus 29,5 ml konzentrierter Salzsäure (32 %-ig) und 140 ml Wasser gelöst. Zu dieser Lösung wurde unter Kühlung im Eisbad eine Lösung von 21,3 g Natriumnitrit in 60 ml Wasser so zugetropft, daß die Temperatur zwischen 10 und 20 °C gehalten wurde. Anschließend wurde das Reaktionsgemisch noch 40 min. ohne Kühlung nachgerührt. Sodann wurden 49 g Zinkpulver zugegeben. Das Reaktionsgemisch wurde auf 15 °C gekühlt. Langsam wurden 177 ml konzentrierte Salzsäure zugetropft, wobei das Reaktionsgemisch auf einer Temperatur von 15-30 °C gehalten wurde.

Nachdem das Zink vollständig reagiert hatte, wurden 0,4 l Äthanol zugesetzt und das Reaktionsgemisch zum Sieden erhitzt. Sodann wurden innerhalb von 30 min. 35,9 g Brenztraubensäureäthylester zugetropft. Nach beendeter Zugabe wurde das Reaktionsgemisch noch 60 min. bei Siedetemperatur gerührt und dann 12 Stunden bei Raumtemperatur stehengelassen. Anschließend wurde der Alkohol unter vermindertem Druck abdestilliert und das zurückbleibende wäßrige Reaktionsgemisch mit Toluol extrahiert. Der Toluolextrakt wurde mit Wasser gewaschen, über Natriumsulfat getrocknet und eingeengt. Der die rohe Titelverbindung enthaltende Rückstand wurde durch Chromatographie an Kieselgel gereinigt. Es wurden 28,7 g der gereinigten Titelverbindung mit einem Schmelzpunkt von 70-72 °C erhalten.

### Beispiel 4:

### 1-Methylindol-2-carbonsäureäthylester.

30 g N-Methylanilin wurden in 300 ml Eisessig gelöst und die Lösung wurde unter Eiskühlung bei einer Temperatur von 10-20 °C mit einer Lösung von 19,9 Natriumnitrit in 75 ml Wasser tropfenweise versetzt. Anschließend wurde 20 min. bei 10-20 °C weitergerührt. Zu der das gebildete N-Nitroso-N-methylanilin enthaltenden Reaktionslösung wurden anschließend unter weiterer Kühlung während ca. 40 min. 75 g Zinkstaub portionsweise gegeben, wobei die Reaktionstemperatur unter 25 °C gehalten wurde. Sodann wurden 720 g N-Salzsäure zu dem Gemisch gegeben und eine Stunde weitergerührt. Das das gebildete N-Amino-N-methylanilin und das Zinksalz enthaltende saure Reaktionsgemisch wurde auf 70 °C erwärmt. Es wurden tropfenweise 34 g Brenztraubensäureäthylester zugesetzt und das Gemisch noch 50 min. bei 70 °C weitergerührt und dann 16 Stunden bei Raumtemperatur stehengelassen. Hierbei wurde das intermediär gebildete Hydrazon sofort in situ zu 1-Methylindol-2-carbonsäureäthylester kondensiert. Zur Aufarbeitung wurde das Reaktionsgemisch dreimal mit insgesamt 1,5 l Toluol extrahiert. Die vereinigten Toluolextrakte wurden zweimal mit insgesamt 500 ml Wasser gewaschen, über Natriumsulfat getrocknet und eingeengt. Es wurden 360 g roher 1-Methylindol-2-carbonsäureäthylester erhalten, welcher durch Chromatographie an Kieselgel unter Verwendung von Dichlormethan als Eluens gereinigt wurde. Das gereinigte Produkt wurde aus Äthanol/Petroläther kristallisiert. Es wurden 27,2 g 1-Methylindol-2-carbonsäureäthylester mit einem Schmelzpunkt von 62-64 °C erhalten.

## Patentansprüche

1. Verfahren zur Herstellung von Indolcarbonsäurederivaten der allgemeinen Formel I 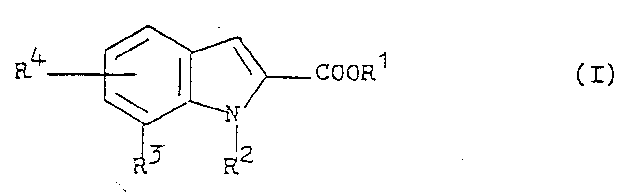 worin
R¹ Wasserstoff oder niederes Alkyl bedeutet,
R² niederes Alkyl oder eine gegebenenfalls durch niederes Alkyl, Halogen oder niederes Alkoxy substituierte carbocyclische Aryl- oder Arylniederalkylgruppe bedeutet, und
R³ Wasserstoff, niederes Alkyl, niederes Alkoxy oder eine gegebenenfalls durch niederes Alkyl, Halogen oder niederes Alkoxy substituierte carbocyclische Aryl- oder Arylniederalkylgruppe bedeutet, oder
R² und R³ gemeinsam eine gegebenenfalls durch niederes Alkyl substituierte Alkylenkette mit 2 bis 4 Kohlenstoffatomen bilden, an welche gegebenenfalls ein 5-6-gliedriger carbocyclischer Ring anelliert sein kann, oder R² und R³ gemeinsam eine -X-CH₂-CH₂-Kette, worin X an den Phenylring des Indolgerüstes gebunden ist und Sauerstoff oder Schwefel bedeutet, bilden, und
R⁴ Wasserstoff, niederes Alkyl, niederes Alkoxy, Halogen oder Trifluormethyl bedeutet,
dadurch gekennzeichnet, daß man
a) Anilinderivate der allgemeinen Formel II 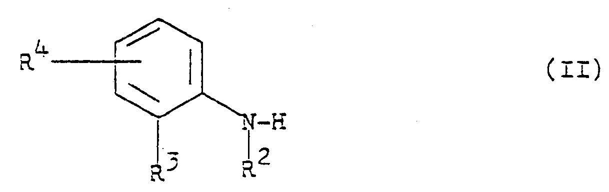 worin R² , R³ und R⁴ obige Bedeutung besitzen, in einer flüssigen organischen Säure oder in einem eine bezogen auf die Verbindung der Formel II mindestens äquivalente Menge einer Säure und gegebenenfalls ein mit Wasser mischbares organisches Lösungsmittel enthaltenden wäßrigen Medium mit Natriumnitrit bei Temperaturen zwischen 0 und 30 °C zu dem entsprechenden Nitrosamin umsetzt,
b) der unter a) erhaltenen nitrosaminhaltigen Reaktionslösung gegebenenfalls unter Zusatz eines mit Wasser mischbaren organischen Lösungsmittels eine bezogen auf die Verbindung der Formel II mindestens 2-fach molare Menge an metallischem Zink zusetzt und gegebenenfalls weitere Säure zusetzt, bis eine zur Reduktion des Nitrosamins zum entsprechenden Hydrazin ausreichende Säuremenge vorliegt, wobei das Zink in die bereits die gesamte Säuremenge enthaltende Lösung oder die Säure nach Zugabe des Zink unter Kühlung so langsam zugesetzt werden, daß in der Reaktionslösung eine Temperatur zwischen 0 und 30 °C eingehalten wird, und das Reaktionsgemisch bei dieser Temperatur für eine zur Reduktion des Nitrosamins zu dem entsprechenden Hydrazin ausreichenden Zeit reagieren läßt, und gegebenenfalls weitere Säure zusetzt, um die Umsetzung eines in dem Reaktionsgemisch vorhandenen Überschuß an metallischem Zink zu vervollständigen,
c) die unter b) erhaltene hydrazinhaltige Reaktionslösung mit einem Brenztraubensäurenniederalkylester bei Temperaturen zwischen Raumtemperatur und 120 °C umsetzt und gewünschtenfalls anschließend die gebildeten Indolcarbonsäureesterderivate der Formel Ia 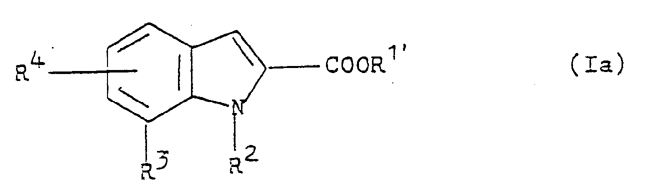 worin R² und R³ obige Bedeutung besitzen und R¹' niederes Alkyl bedeutet, zu der freien Säure hydrolysiert.

2. Verfahren nach Anspruch 1, dadurch gekennzeichnet, daß man als Säuren Essigsäure und/oder wäßrige Salzsäurelösung einsetzt.

3. Verfahren nach Anspruch 1, dadurch gekennzeichnet, daß man Verbindungen der Formel II einsetzt, worin R² und R³ gemeinsam eine gegebenenfalls durch niederes Alkyl substituierte Alkylenkette mit 2 bis 4 Kohlenstoffatomen bilden.

4. Verfahren nach Anspruch 1, dadurch gekennzeichnet, daß man als mit Wasser mischbares organisches Lösungsmittel einen niederen Alkohol einsetzt.

5. Verfahren nach Anspruch 1, dadurch gekennzeichnet, daß das Natriumnitrit in einer bezogen auf die Verbindung der Formel II äquimolaren Menge eingesetzt wird.

6. Verfahren nach Anspruch 1, dadurch gekennzeichnet, daß in Schritt b in der Reaktionslösung eine Säuremenge von mindestens 5 Äquivalenten Säure bezogen auf die in Schritt a eingesetzte Verbindung der Formel II vorliegt.

## Claims

1. A method for the preparation of indole carboxylic acid derivatives of the general Formula I, 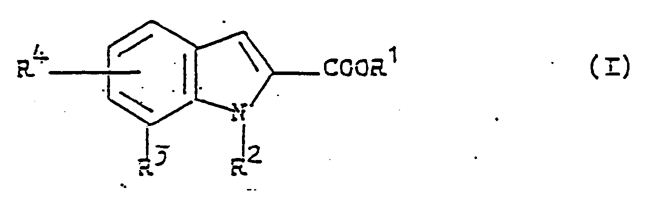 wherein
R¹ is hydrogen or lower alkyl,
R² is lower alkyl or a carbocyclic aryl group or carbocyclic aryl lower alkyl group, which groups may optionally be substituted by lower alkyl, halogen or lower alkoxy, and
R³ is hydrogen, lower alkyl, lower alkoxy or a carbocyclic aryl group or carbocyclic aryl lower alkyl group, which groups may optionally be substituted by lower alkyl, halogen or lower alkoxy, or
R² and R³ together form an alkylene chain with 2 to 4 carbon atoms which may optionally be substituted by lower alkyl, and to which a 5-6-member carbocyclic ring may optionally be fused, or R² and R³ together form an -X-CH₂-CH₂- chain, wherein X is bonded to the phenyl ring of the indole structure and is oxygen or sulphur, and
R⁴ is hydrogen, lower alkyl, lower alkoxy, halogen or trifluoromethyl,
characterised in that
a) aniline derivatives of the general Formula II, 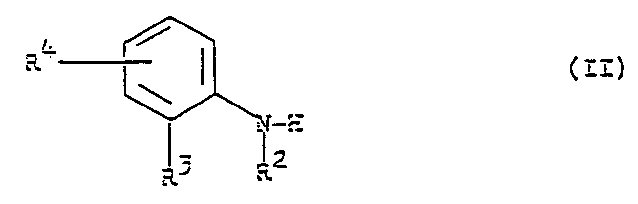 wherein R², R³ and R⁴ have the above meanings, are reacted with sodium nitrite at temperatures of between 0 and 30°C in a liquid organic acid or in an aqueous medium containing a quantity of an acid at least equivalent to the compound of Formula II and optionally a water-miscible organic solvent, to produce the corresponding nitrosamine,
b) an at least twice molar quantity of metallic zinc, relative to the compound of Formula II, is added to the nitrosamine-containing reaction solution obtained under a), optionally with the addition of an organic solvent which is miscible with water, and optionally additional acid is added until there is a quantity of acid sufficient for the reduction of the nitrosamine to the corresponding hydrazine, whereby the zinc is added to the solution which already contains the entire quantity of acid, or after the addition of the zinc, the acid is added with cooling so slowly that a temperature of between 0 and 30°C is maintained in the reaction solution, and the reaction mixture is allowed to react at this temperature for a time sufficient for the reduction of the nitrosamine to the corresponding hydrazine, and optionally additional acid is added in order to complete the reaction of an excess of metallic zinc which is present in the reaction mixture,
c) the hydrazine-containing reaction solution obtained under b) is reacted with a pyruvic acid lower alkyl ester at temperatures between room temperature and 120°C and if desired subsequently the resulting indole carboxylic acid ester derivatives of Formula Ia, 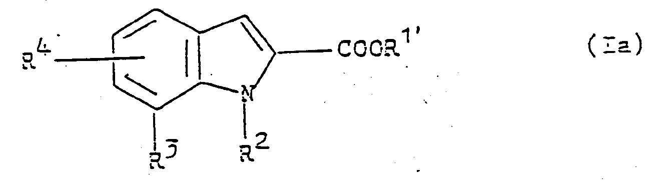 wherein R² and R³ have the above meanings and R^{1'} is lower alkyl, are hydrolysed to produce the free acid.

2. A method according to Claim 1, characterised in that acetic acid and/or aqueous hydrochloric acid solution are used as acids.

3. A method according to Claim 1, characterised in that compounds of Formula II are used wherein R² and R³ together form an alkylene chain having 2 to 4 carbon atoms and optionally substituted by lower alkyl.

4. A method according to Claim 1, characterised in that a lower alcohol is used as the water-miscible organic solvent.

5. A method according to Claim 1, characterised in that the sodium nitrite is used in a quantity which is equimolar relative to the compound of Formula II.

6. A method according to Claim 1, characterised in that in step b) an acid quantity of at least 5 equivalents acid relative to the compound of Formula II used in step a) is present in the reaction solution.

## Revendications

1. Procédé de préparation de dérivés de l'acide indolecarboxylique, de formule générale I 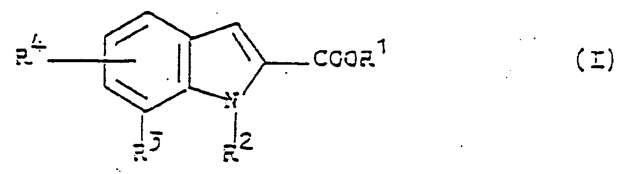 dans laquelle :
R¹ représente un atome d'hydrogène ou un groupe alkyle inférieur ;
R² représente un groupe alkyle inférieur ou un groupe carbocyclique aryle ou aryl-alkyle(inférieur), éventuellement substitué par un groupe alkyle inférieur, halogène ou alcoxy inférieur, et
R³ représente un atome d'hydrogène, un groupe alkyle inférieur, alcoxy inférieur ou un groupe carbocyclique aryle ou aryl-alkyle (inférieur) éventuellement substitué par un groupe alkyle inférieur, halogène ou alcoxy inférieur, ou bien
R² et R³ forment ensemble une chaîne alkylène de 2 à 4 atomes de carbone éventuellement substituée par un groupe alkyle inférieur sur laquelle peut éventuellement être condensé un noyau carbocyclique pentagonal ou hexagonal, ou bien R² et R³ forment ensemble une chaîne -X-CH₂-CH₂-, dont X est fixé sur le noyau phényle du squelette de l'indole et représente un atome d'oxygène ou de soufre, et
R4 représente un atome d'hydrogène, un groupe alkyle inférieur alcoxy inférieur, halogéno ou trifluorométhyle, procédé caractérisé en ce que :
(a) on fait réagir des dérivés de l'aniline, de formule générale II 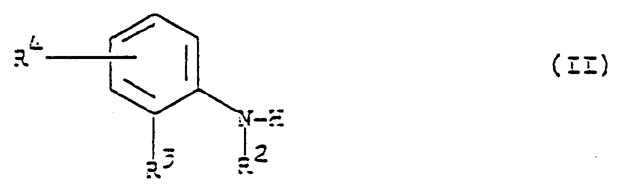 dans laquelle R², R³ et R⁴ ont le sens ci-dessus, dans un acide organique liquide ou dans un milieu aqueux contenant une quantité au moins équivalente, (par rapport au composé de formule II) d'un acide et éventuellement un solvant organique miscible à l'eau, avec du nitrite de sodium à des températures comprises entre 0 et 30°C, pour obtenir la nitrosamine correspondante :
(b) à la solution réactionnelle, contenant de la nitrosamine, obtenue en (a), et éventuellement .avec addition d'un solvant organique miscible à l'eau, on ajoute une quantité molaire au moins double (par rapport au composé de formule II) de zinc métallique et éventuellement un supplément d'acide jusqu'à présence d'une quantité d'acide suffisante pour la réduction de la nitrosamine en hydrazine correspondante en ajoutant le zinc dans la solution contenant déjà la quantité totale d'acide ou bien en ajoutant l'acide, après addition du zinc, sous refroidissement, si lentement que l'on maintient dans la solution réactionnelle une température comprise entre 0 et 30°C et on laisse réagir à cette température pendant une période de temps suffisante pour réaliser la réduction de la nitrosamine en hydrazine correspondante, et éventuellement on ajoute un supplément d'acide pour parachever la réaction d'un excès de zinc métallique présent dans le mélange réactionnel ;
(c) on fait réagir, à des températures entre la température ambiante et 120°C la solution réactionnelle, contenant de l'hydrazine obtenue en (b), avec un ester alkylique d'acide pyrotartrique et, si on le désire, on soumet ensuite les dérivés de type ester d'acide indolecarboxylique de formule (Ia) 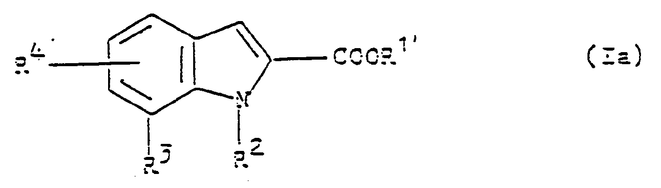 dans laquelle R² et R³ ont le sens précité et R^{1'} signifie un alkyle inférieur à une hydrolyse donnant l'acide libre.

2. Procédé selon la revendication 1, caractérisé en ce qu'on utilise comme acides l'acide acétique et/ou une solution aqueuse d'acide chlorhydrique.

3. Procédé selon la revendication 1, caractérisé en ce qu'on utilise des composés de formule II, dans laquelle R² et R³ forment ensemble une chaîne alkylène, éventuellement substituée par un groupe alkyle inférieur, et comportant 2 à 4 atomes de carbone.

4. Procédé selon la revendication 1, caractérisé en ce qu'on utilise un alcool inférieur comme solvant organique miscible à l'eau.

5. Procédé selon la revendication 1, caractérisé en ce qu'on utilise le nitrite de sodium en une quantité équimolaire par rapport au composé de formule II.

6. Procédé selon la revendication 1, caractérisé en ce que, dans l'étape (b), il y a dans la solution de réaction une quantité d'acide représentant au moins cinq équivalents d'acide par rapport au composé de formule II utilisé dans l'étape (a).
